# EUROPEAN PATENT APPLICATION

(11) **EP 4 817 510 A1**
(43) Date of publication of application: **07.10.2026**
(21) Application number: 25382319.9
(22) Date of filing: 31.03.2025
(51) Int. Cl.: A61B 5/00, A61B 5/02, A61B 5/0215, A61B 5/026

(54) **IMAGING SYSTEM TO CALCULATE HAEMODYNAMIC PARAMETERS AND COMPUTER PROGRAM AND METHOD THEREOF**

(71) Applicant: Gutiérrez-Chico, Juan Luis, 44263 Dortmund (DE)
(72) Inventor: Gutiérrez-Chico, Juan Luis, 44263 Dortmund (DE)
(74) Representative: Hoffmann Eitle

(57) **Abstract**

The present invention relates to an Optical Coherence Tomography (OCT) system for assessing haemodynamic parameters in blood vessels. The system comprises an OCT probe configured to be placed inside a blood vessel and a processing unit in communication with the probe. The processing unit is designed to detect the time intervals corresponding to the passage of a column of an OCT-detectable liquid (ODL) at a reference point in the probe, calculate the relative movement time of the ODL column, and derive haemodynamic parameters such as the coronary microvascular function, absolute coronary flow, coronary flow reserve (CFR), and microvascular resistance, among other possibilities. The system may operate in stationary mode or with controlled pullback speeds of the probe, allowing an improved precision in coronary microvascular function determination. The system also may operate under hyperaemia conditions or under normal conditions. In some embodiments the invention also provides systems and methods for calculating coronary flow and microvascular resistance by integrating imaging and transit time data.

## Description

### Technical field of the invention

The present invention belongs to the field of blood vessel imaging and physiological assessment technologies. Specifically, it relates to imaging systems such as optical coherence tomography (OCT) systems and methods for assessing haemodynamic parameters within blood vessels. More precisely, this invention provides a novel OCT-based approach for the evaluation of coronary microvascular function.

### Background of the invention

Coronary microvascular dysfunction (CMD) is increasingly recognized as a significant contributor to cardiovascular disease and a driver of adverse outcomes, even in the absence of obstructive coronary artery disease. Patients with CMD often experience ischemic symptoms, such as angina, due to impaired microvascular function, which can result in misdiagnosis or delayed treatment. Historically, these patients were dismissed with a reassuring but misleading conclusion that their symptoms were unrelated to the heart, often leading to prolonged suffering and increased morbidity. CMD is now understood to adversely affect both quality of life and long-term prognosis, necessitating accurate and accessible methods for its diagnosis and management.

Coronary flow reserve (CFR) and microvascular resistance are two key parameters used to evaluate coronary microvascular function. CFR measures the capacity of coronary blood flow to increase in response to stress, while microvascular resistance directly reflects the functional state of the coronary microcirculation. Accurate measurement of these parameters is critical for identifying CMD and tailoring appropriate treatments.

Currently, the assessment of coronary microvascular function relies primarily on two methods: thermodilution and Doppler wire-based measurements. The Doppler wire technique employs an intracoronary wire with a Doppler transducer to measure blood velocity and derive CFR. However, this method is costly, requires specialized equipment, and demands technical expertise for operation and interpretation. Consequently, its use has been limited to specialized centres, and its availability is restricted.

Thermodilution, now more commonly employed due to advancements in sensor technology, is based on the principle of indicator-dilution. It involves the injection of saline at a temperature different from blood and measuring temperature changes downstream to calculate parameters such as transit time and resistance. This method is less dependent on specialized wires but introduces its own limitations. When performed with a bolus injection of saline, the absolute coronary flow cannot be calculated, as the volume of infused saline is not precisely controlled due to spillover into the aorta. Continuous infusion techniques allow the measurement of absolute coronary flow, but require dedicated and expensive catheters, such as RayFlow, which are not routinely available in all catheterization laboratories. This adds cost and complexity to the procedure, limiting its widespread adoption.

The limitations of these techniques underscore the need for alternative methods that are both accurate and practical for routine clinical use. Current approaches fail to integrate seamlessly into standard catheterization workflows, often requiring additional equipment or specialized training. Moreover, they do not leverage technologies already widely available in many catheterization laboratories, and require specialized, often expensive equipment and consumables.

Thus, there is a need for a method and system that can assess coronary microvascular function in a simpler, faster, and more cost-effective manner. Such a solution should integrate with existing catheterization lab equipment, minimize the need for additional tools, and overcome the limitations of thermodilution and Doppler wire methods. This would allow generalizing CMD diagnostics and, thus, improving patient outcomes.

### Summary of the invention

In a first aspect of the invention, an Optical Coherence Tomography (OCT) system comprising an OCT probe and a processing unit in communication with a memory and with the OCT probe is disclosed, wherein the OCT probe is configured to be placed inside a blood vessel, and the memory comprises instructions that, when read by the processing unit, cause said processing unit to: detect the time *t*₀ at which the distal end of a first travelling column of an OCT-detectable liquid (ODL) of volume *V_{c}* travelling inside the blood vessel towards the OCT probe tip at a velocity *v_{c}* reaches a reference point in the OCT probe, and the time *t*₁ at which the proximal end of the first travelling column of ODL reaches said reference point in the OCT; calculate a relative-movement time *T*₁ of the ODL column with respect to the reference point of the OCT probe as a function of *t*₁ and *t*₀, preferably as the difference between *t*₁ and *t*₀; and calculate one or more haemodynamic parameters associated to the blood vessel as a function of *T*₁, preferably wherein said haemodynamic parameters comprise the absolute coronary flow, the coronary flow reserve and/or the microvascular resistance.

According to a preferred embodiment of the first aspect of the invention, the OCT probe is configured to remain stationary inside the blood vessel during the travelling of the first ODL column, and the memory comprises further instructions which, when executed by the processing unit, cause said processing unit to: calculate the absolute coronary flow Q₁ as a function of *V_{c}* and the relative-movement time *T*₁.

According to a preferred embodiment of the first aspect of the invention, the memory comprises further instructions which, when executed by the processing unit, cause said processing unit to: perform one or more previous steps in hyperaemia conditions of the vessel to calculate a hyperaemia relative-movement time *T*_{1*H*} of the ODL column with respect to the OCT probe; and estimate a coronary flow reserve (CFR) parameter as a function of *T*₁ and *T*₁*_{H},* preferably as the ratio between *T*_{1*H*} and *T*₁.

According to a preferred embodiment of the first aspect of the invention, the probe is further configured to be displaced at a pullback speed *PBs*₁ when the first column of ODL is travelling inside the vessel, and at a pullback speed *PBs*₂*,* different than *PBs*₁, when a second column of ODL of volume *V_{c}* is travelling inside the vessel towards the OCT probe tip at a velocity *v_{c}*, wherein the volume *V_{c}* and the velocity *v_{c}* of the second column of ODL are the same or approximately the same as those of the first column of ODL, and wherein the memory comprises further instructions which, when executed by the processing unit, cause said processing unit to: optionally, calculate a first probe displacement length ${l}_{1}^{′}$ as a function of *T*₁ and *PBs*₁; detecting the time ${t}_{0}^{′}$ at which the distal end of the second travelling column of ODL reaches a reference point in the OCT probe, and the time ${t}_{1}^{′}$ at which the proximal end of the travelling column of ODL reaches said reference point in the OCT; calculate a relative-movement time *T*₂ of said second column of ODL (2) with respect to the OCT probe reference point as a function of ${t}_{0}^{′}$ and ${t}_{1}^{′}$*,* and optionally calculate a second probe displacement length ${l}_{2}^{′}$ as a function of *T*₂ and *PBs₂*; calculate the ODL column velocity *v_{c}* and/or the column length *l_{c},* as a function of *T*₁, *T*₂*, PBs*₁ and *PBs₂,* or as a function of ${l}_{1}^{′} , {l}_{2}^{′}$, *PBs*₁ and *PBs₂*; and calculate one or more haemodynamic parameters associated to the blood vessel as a function of *v_{c}* and/or *l_{c},* preferably wherein said haemodynamic parameters are the absolute coronary flow, the coronary flow reserve and/or the microvascular resistance.

According to a preferred embodiment of the first aspect of the invention, the memory comprises further instructions which, when executed by the processing unit, cause said processing unit to: calculate the ODL column velocity in hyperaemia conditions *v_{cH}*; and calculate the coronary flow reserve CFR as a function of *v_{cH}* and *v_{c}*, preferably as the ratio between *v_{cH}* and *v_{c}.*

According to a preferred embodiment of the first aspect of the invention, wherein the memory comprises further instructions which, when executed by the processing unit, cause said processing unit to calculate the absolute coronary flows *Q*₁ and/or *Q*₂ of the first and second ODL columns respectively, as a function of the volume *V_{c}* and the transit time *TT*₁ and/or *TT*₂ of the first and second ODL columns respectively, wherein *TT*₁ is calculated as a function of *PBs*₁, *v_{c}* and *T*₁, and *TT*₂ is calculated as a function of *PBs*₂*, v_{c}* and *T*₂.

According to a preferred embodiment of the first aspect of the invention, wherein the memory comprises further instructions which, when executed by the processing unit, cause said processing unit to: image with the OCT probe the blood vessel and estimate the pressure P_{d} at a distal point of the vessel by means of computational physiology, preferably by optical flow ratio (OFR); and calculate a microvascular resistance parameter Rₘ as a function of the pressure P_{d} and the absolute coronary flow *Q*₁ or *Q*₂, or as a function of the pressure P_{d} and the transit time *TT*₁ or *TT*₂*.*

According to a preferred embodiment of the first aspect of the invention, the OCT system further comprises, or is further configured to be connected to, an automatic injector of ODL, able to infuse a column of known volume *V_{c}* of ODL at a constant flow rate (*Qᵣ*) during a predetermined infusion time (*Tᵢ*).

According to a preferred embodiment of the first aspect of the invention, the OCT system further comprises a guide-extension catheter to inject the ODL column.

In a second aspect of the invention, a computer program is disclosed, the computer comprising instructions which, when executed by a processor, causes the processor to carry out the following steps: detecting the time *t*₀ at which the distal end of a first travelling column of ODL of known volume *V_{c}* travelling at a velocity *v_{c}* towards the tip of an OCT probe placed inside a blood vessel, reaches a reference point in the OCT probe, and the time *t*₁ at which the proximal end of the first travelling column of ODL reaches said reference point in the OCT; calculating a relative-movement time *T*₁ of the ODL column with respect to the reference point of the OCT probe as a function of *t*₀ and *t*₁, preferably as the difference between *t*₁ and *t*₀; calculating one or more haemodynamic parameters associated to the blood vessel as a function of *T*₁, preferably wherein said haemodynamic parameters comprise the absolute coronary flow, the coronary flow reserve and/or the microvascular resistance, more preferably the haemodynamic parameters comprise the absolute coronary flow Q₁ calculated as a function of *V_{c}* and the relative-movement time *T*₁.

According to a preferred embodiment of the second aspect of the invention, the computer program comprises further instructions which, when executed by a processor, causes the processor to further carry out the following steps: estimating a coronary flow reserve (CFR) parameter as a function of *T*₁ and *T*₁*_{H},* preferably as the ratio between *T*_{1*H*} and *T*₁, wherein *T*_{1*H*} is a hyperaemia relative-movement time *T*_{1*H*} of the ODL column with respect to the reference point of the OCT probe, obtained performing steps (i) to (iv) in hyperaemia conditions of the vessel.

According to a preferred embodiment of the second aspect of the invention, the computer program comprises further instructions which, when executed by a processor, causes the processor to carry out the following steps: detecting the times *t*₀ and ${t}_{0}^{′}$, respectively, at which the distal end of the first and a second columns of ODL both having each a volume *V_{c}* and both travelling inside a blood vessel at a velocity *v_{c}*, reach the reference point in the OCT probe, and the times *t*₁ and ${t}_{1}^{′}$ at which the proximal end of the first and second travelling columns of ODL, respectively, reach said reference point in the OCT, wherein the OCT probe is moving at a pullback speed *PBs*₁ with respect to the first column of ODL and at a pullback speed *PBs*₂ with respect to the second column of ODL; calculating relative-movement times *T*₁ and *T*₂ of the first and second ODL columns, respectively, with respect to the reference point of the OCT probe as a function of *t*₀ and *t*₁, and as a function of ${t}_{0}^{′}$ and ${t}_{1}^{′}$, respectively, preferably as the difference between *t*₁ and *t*₀ and between ${t}_{1}^{′}$ and ${t}_{0}^{′}$; optionally, calculating a first probe displacement length ${l}_{1}^{′}$ as a function of *T*₁ and *PBs*₁, and a second probe displacement length ${l}_{2}^{′}$ as a function of *T*₂ and *PBs₂*; and calculating the ODL column velocity *v_{c}* as a function of *T*₁, *T*₂*, PBs*₁ and *PBs₂,* or as a function of ${l}_{1}^{′} , {l}_{2}^{′}$*, PBs*₁ and *PBs₂,* wherein the ODL column velocity *v_{c}* is related to the blood flow rate.

According to a preferred embodiment of the second aspect of the invention, the computer program comprises further instructions which, when executed by a processor, causes the processor to further carry out the following steps: calculating ODL column velocity in hyperaemia conditions *v_{cH}*; and calculating the coronary flow reserve CFR as a function of *v_{cH}* and *v_{c}*, preferably as the ratio between *v_{cH}* and *v_{c}.*

According to a preferred embodiment of the second aspect of the invention, the computer program comprises further instructions which, when executed by a processor, causes the processor to further carry out the following steps: calculating the absolute coronary flows *Q*₁ and/or *Q*₂ as a function of *V_{c}* and the transit time *TT*₁ of the first ODL column for *Q*₁ or the transit time *TT*₂ of the second ODL column for *Q*₂, wherein *TT*₁ is calculated as a function of *PBs*₁, *v_{c}* and *T*₁, and *TT*₂ is calculated as a function of *PBs*₂*, v_{c}* and *T*₂.

According to a preferred embodiment of the second aspect of the invention, the computer program comprises further instructions which, when executed by a processor, causes the processor to further carry out the following steps: imaging with the OCT probe the blood vessel and estimate the pressure P_{d} at a distal point of the vessel by means of computational physiology, preferably by optical flow ratio (OFR); and calculating a microvascular resistance parameter Rₘ as a function of the pressure P_{d} and the absolute coronary flow *Q*₁ or *Q*₂, or as a function of the pressure P_{d} and the transit time *TT*₁ or *TT*₂*.*

### Brief description of the drawings

To enable a better understanding of the present disclosure, and to show how the present disclosure may be carried out, reference will now be made, by way of example only, to the accompanying schematic drawings, wherein:

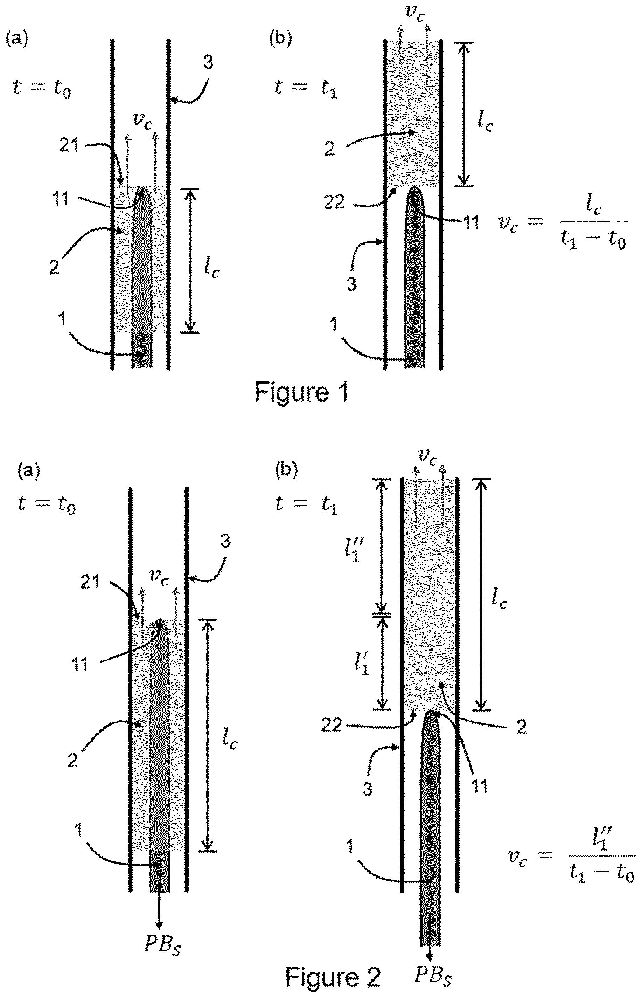

Figure 1 shows an example of an Optical Coherence Tomography (OCT) system according to one or more embodiments of the invention, wherein the OCT probe is stationary inside a blood vessel, wherein (a) shows a distal end of a travelling column of OCT detectable liquid (ODL) reaching the distal end of the OCT probe, wherein an optical scanner is placed, and (b) shows a proximal end of a travelling column of OCT detectable liquid (ODL) reaching said distal end or optical scanner of the OCT probe.
Figure 2 shows an example of an Optical Coherence Tomography (OCT) system according to one or more embodiments of the invention, wherein the OCT probe is being displaced at a pullback speed inside a blood vessel, wherein (a) shows a distal end of a travelling column of OCT detectable liquid (ODL) reaching the distal end of the OCT probe, wherein an optical scanner is placed, and (b) shows a proximal end of a travelling column of OCT detectable liquid (ODL) reaching said distal end or optical scanner of the OCT probe.

### Description of the invention

### Definitions

As used herein, "a" or "an" means "at least one" or "one or more."

It is noted that the term "about", if used herein, refers to +/- 30%, preferably +/- 20%, preferably +/- 15%, more preferably +/- 10%, of the indicated referred value. It may also refer to the exact value, that is, +/- 0% or +/- the standard measurement error. Also, any quantity, composition percentages and/or dimension mentioned herein is understood to present an error of +/- 30%, preferably +/- 20%, preferably +/- 15%, more preferably +/- 10%, more preferably +/- 5% or +/-0% of the indicated value. It may also refer to the exact value, that is, +/- 0% or +/- the standard measurement error.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together.

When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

Unless defined otherwise, all technical and scientific terms used herein have the same meaning as is commonly understood by one of ordinary skill in the art to which this invention belongs. All patents, applications, published applications and other publications referred to herein are incorporated by reference in their entirety. If a definition set forth in this section is contrary to or otherwise inconsistent with a definition set forth in applications, published applications and other publications that are herein incorporated by reference, the definition set forth in this section prevails over the definition that is incorporated herein by reference.

The term "Optical Coherence Tomography (OCT) system", in the context of the invention, refers to an invasive imaging technology based on the interference of light waves to create detailed cross-sectional images of internal structures. In the context of the invention, the OCT system comprises an OCT probe and a processing unit in communication with the probe. In some embodiments the system may also comprise additional components, such as an automatic injector for infusing a liquid, a guide-extension catheter, and computational tools for analysing haemodynamic parameters. Typically, an OCT system can be employed for imaging biological tissues, whereas in this invention it is used for determining physiological or functional characteristics, such as those related to haemodynamic parameters within blood vessels.

The term "OCT probe", in the context of the invention, refers to a device or component of the OCT system designed to be inserted into a blood vessel to capture optical data. The OCT probe in some embodiments may comprise a light source, optical fibres for delivering and collecting light, and imaging elements, such as a camera or sensor. The probe may be configured to remain stationary or move within the vessel as required by the application, and it allows real-time visualization and data acquisition for assessing the structure and/or, within the context of the invention, flow within the vessel. Examples of configurations include probes optimized for coronary arteries, microvascular structures, or other blood vessels, with dimensions and materials suitable for intracoronary or general intravascular use.

The term "OCT probe tip", in the context of the invention, refers to the distal end of the OCT probe. In the context of the invention, the OCT probe tip is distal to the ODL column injection place, therefore the ODL travels towards said OCT probe tip in a first instance. The OCT probe tip may house optical and imaging components, such as a camera or sensor, light delivery ports, and/or detectors. In some embodiments it may serve as a reference point for detecting and calculating parameters associated with the movement of the liquid. The design of the tip may vary depending on the intended application, including shapes and sizes optimized for specific vessel types.

The term "a reference point in the OCT probe", in the context of the invention, refers to a landmark point or location, real or virtual, which may be placed within or on the OCT probe and used as a basis for detecting the movement of the OCT-detectable liquid (ODL) relative to the movement of the OCT probe. This reference point in some embodiments may coincide with a sensor of the OCT probe, for example with a sensor able to detect the presence of ODL, therefore being able to detect the arrival and exit of the ODL column, or it may coincide with the OCT probe tip, a point along the length of the probe, or an element of the imaging system, such as the camera or light source. For example, the reference point may be defined as the distal end of the OCT probe tip. Alternatively, it may be an image-capturing or data-capturing position, such as the position of the camera or sensor. The reference point in some embodiments could be considered virtual when the presence of the ODL is detected when optical or video data changes due to the interaction with the ODL (e.g., a change in color, clarity, or pattern in the sensor or image data retrieved). In some embodiments it may also refer to a specific marker or surface on the probe, a geometrical feature, or a computationally derived point used for processing the interaction of light waves with the liquid or the vessel. The reference point may also be understood as an abstract point employed to measure the time or movement of the ODL column relative to the movement of the probe, and it may not be a physical point but estimated through changes detected in the sensors of the camera related to movement of the ODL column.

The term "blood vessel", in the context of the invention, refers to any tubular structure in the human or animal body through which blood flows, including arteries, veins, capillaries, and microvascular networks. Preferably, it refers to coronary vessels, and more particularly to coronary microvasculature. These structures may vary in size, flow dynamics, and physiological properties, encompassing both healthy and diseased vessels, as well as those subject to medical interventions.

The term "OCT detectable liquid (ODL)", in the context of the invention, refers to any liquid that can be visualized, detected or distinguished using optical coherence tomography imaging. This liquid may comprise, for example, transparent fluids, saline solutions, contrast agents, or other biocompatible fluids with optical properties suitable for OCT detection. In some embodiments it may comprise an isotonic saline solution, a high-viscosity contrast medium, or a solution containing optically active substances. The liquid is preferably safe for intravascular use, biocompatible, and/or sterile.

The term "travelling column of an OCT detectable liquid", in the context of the invention, refers to an injected volume of ODL moving within the blood vessel. For computational purposes, the column may be described as having a distal end, which leads its movement, and a proximal end, which trails it. However, in some embodiments, the column may not have sharply defined boundaries and may instead exhibit a gradient of concentration. The criteria for defining the proximal and distal ends of the column may involve a predetermined concentration threshold of the OCT detectable liquid, distinguishing it from the surrounding blood or other fluids.

The term "distal end of a first travelling column of an OCT detectable liquid", in the context of the invention, refers to the leading edge of the moving column as it travels through the blood vessel towards the OCT probe tip. This end is, thus, the first portion of the column to reach the reference point or the sensor of the OCT probe.

The term "proximal end of a first travelling column of an OCT detectable liquid", in the context of the invention, refers to the trailing edge or the rear portion of the column as it travels towards the OCT probe tip. It is, thus, the last part of the column to pass through the reference point or sensor of the OCT probe.

The term "travelling column velocity", in the context of the invention, refers to the speed at which the column of OCT detectable liquid moves through the blood vessel. This velocity is determined as a function of the relative position of the proximal and distal ends of the column over time and in the context of this invention is calculated using OCT imaging data. For calculation purposes, in some embodiments it may be assumed that all the liquid inside the column moves at the same velocity or at an average velocity.

The term "relative-movement time", in the context of the invention, refers to the time duration during which the column of OCT detectable liquid interacts with or contacts with the reference point or the sensor of the OCT probe. Specifically, it represents the interval from the arrival of the distal end of the travelling column at the reference point to the exit of the proximal end, while the travelling column is moving relative to the OCT probe. In some embodiments the relative movement comprises probe movement as well, preferably in a direction opposite to the direction of movement of the ODL column.

The term "haemodynamic parameters associated to the blood vessel", in the context of the invention, refers to measurable or calculable characteristics that describe the dynamics of blood flow and vascular function, such as microvascular function. These parameters include, but are not limited to, absolute coronary flow, coronary flow reserve (CFR), and microvascular resistance. Additionally, in some embodiments the term may encompass any parameter that can be derived from the calculation of the liquid column transit time, the volume of injected liquid, or the length of the liquid column, such as, for example, vascular leakage, flow velocity, or vascular compliance.

The term "absolute coronary flow", in the context of the invention, refers to the volume of blood flowing through a coronary artery per unit of time, typically expressed in millilitres per minute (mL/min). This parameter provides a direct measure of epicardial coronary circulation and can be calculated using the known volume of an OCT detectable liquid column, its transit time, and, preferably, the characteristics of the vessel. Absolute coronary flow may be used as an estimator of both epicardial and microvascular function, in combination with other physiology parameters.

The term "coronary flow reserve", in the context of the invention, refers to the ratio of the coronary blood flow at maximal hyperaemia to the blood flow under resting conditions. This parameter indicates the capacity of the coronary circulation to increase blood flow in response to stress or increased myocardial demand. A reduced CFR is indicative of impaired microvascular or epicardial function and is used to diagnose conditions such as coronary microvascular dysfunction.

The term "microvascular resistance", in the context of the invention, refers to the resistance to blood flow within the coronary microvasculature, and it is determined by the interplay between blood viscosity, vessel diameter, and vascular tone. This parameter is often expressed in units such as Wood units (mmHg·min/L) or dyn·s·cm⁻⁵ and can be derived using the Ohm's law analogy for the cardiovascular system, where resistance is calculated as the ratio of pressure difference across the microvascular bed to the flow rate. Microvascular resistance provides insight into the functional state of the coronary microvasculature and is useful for diagnosing microvascular dysfunction.

The term "hyperaemia conditions", in the context of the invention, refers to a physiological state in which blood vessels are dilated, resulting in increased blood flow through the vessel. Hyperaemia conditions can be induced pharmacologically, for example, by administering agents such as adenosine or papaverine. In the context of the invention, unless otherwise specified, measurements are understood to be taken under normal conditions, which represent resting physiological states with basal coronary blood flow. Hyperaemia conditions are therefore different conditions than these resting states and may be used to, among other options, assess the maximum functional capacity of the coronary vasculature, such as in determining parameters like coronary flow reserve.

The term "pullback speed", in the context of the invention, refers to the rate at which the OCT probe is retracted within the blood vessel during imaging or measurement procedures conducted in pullback mode. Pullback mode is a standard technique employed in many cardiology practices to enable the systematic imaging of the vessel's interior over its length, typically used to create longitudinal profiles, or, in the context of this invention, to analyse flow dynamics. The pullback speed can be expressed as the distance the probe is retracted over a defined time interval (e.g., millimeters per second). This speed is adjustable and may be predetermined based on the specific diagnostic requirements. It is understood that if the probe remains stationary, the pullback speed is considered to be zero.

The term "probe displacement length", in the context of the invention, refers to the distance travelled by the OCT probe during its movement in pullback mode within the blood vessel. This displacement occurs as the probe is retracted over a measured distance or a quantity of time, typically for the purpose of capturing imaging data or, in the context of this invention, performing calculations related to flow dynamics or vessel structure.

The term "computational physiology", in the context of the invention, refers to the application of computational models, algorithms, and techniques to simulate, analyse, or interpret physiological functions. This includes, but is not limited to, methods like optical flow ratio (OFR) and other advanced computational approaches used to estimate physiological parameters such as pressure, flow dynamics, or vascular resistance.

The term "optical flow ratio", in the context of the invention, refers to a computational method used to estimate the pressure gradient and flow characteristics within a blood vessel based on OCT imaging data. This technique relies on reconstructing the vessel geometry and analysing the optical flow of blood or an injected liquid to derive physiological parameters such as pressure drop or flow reserve.

The term "guide-extension catheter", in the context of the invention, refers to a catheter used to extend the reach of the guide catheter into a target blood vessel to facilitate the delivery of other instruments, such as an OCT probe or the injection of OCT-detectable liquids. Guide-extension catheters are preferably flexible, tubular devices that provide a stable pathway for precise instrument placement or liquid delivery in challenging anatomical scenarios. It is noted that these catheters are commonly employed in cardiology practices for procedures involving complex or distal coronary lesions.

### Description

Coronary microvascular dysfunction (CMD) is a significant contributor to cardiovascular morbidity and mortality, often undiagnosed or misdiagnosed due to the limitations of current diagnostic tools. Accurate assessment of coronary microvascular function, which is of great importance for effective diagnosis and management, relies on measuring parameters such as coronary flow reserve (CFR) and microvascular resistance, among others. However, existing methods to obtain the microvascular function such as Doppler wire and thermodilution present considerable drawbacks. For instance, the Doppler wire technique is costly, requires specialized equipment, and demands significant technical expertise, limiting its accessibility. Thermodilution, on the other hand, while more commonly used, involves complex setups, expensive consumables, and inherent methodological limitations.

This invention introduces a novel approach to assessing coronary microvascular function, leveraging widely available imaging technologies, such as Optical Coherence Tomography (OCT), in an innovative and practical manner. Unlike conventional methods, the imaging system and method of the invention, preferably an OCT-based system, enables the evaluation of haemodynamic parameters such as CFR, absolute coronary flow, and microvascular resistance, among others, using off-the-shelf OCT imaging equipment. By incorporating a column of an OCT-detectable liquid (ODL), this system allows to utilize the relationship between transit time, column length and/or flow velocity to deliver precise and comprehensive assessments of coronary microvascular function. This method addresses the limitations of existing techniques by eliminating the need for specialized wires or complex equipment and integrating seamlessly with standard catheterization workflows.

The system of the invention comprises an imaging system, preferably an OCT apparatus that includes an OCT probe and a processing unit, wherein the OCT probe is designed to be deployed within a blood vessel, preferably a coronary artery or coronary microvasculature. OCT, a widely used intracoronary imaging system, is primarily used for assessing coronary anatomy, guiding percutaneous coronary interventions, and characterizing plaque morphology. Additionally, it is routinely used in many catheterization laboratories, and it is widely available out-of-the-shelve. However, currently OCT systems are not utilized to measure haemodynamic parameters such as CFR or microvascular resistance, leaving a gap in leveraging this widely available technology for comprehensive coronary assessment. Herein it is described for the first time an OCT probe configured for detecting the transit time, the length, and/or the velocity of a column of ODL as it passes through the vessel, allowing the processing unit of the OCT system to calculate relevant haemodynamic parameters based on this data, such as, but not limited to, absolute coronary flow, CFR, and microvascular resistance. In some embodiments, the system may also comprise an automatic injector for precise delivery of the ODL and a guide-extension catheter to facilitate access to specific vessel locations.

In some embodiments of the invention, the system may calculate the time employed by the ODL column to pass over the OCT probe (herein referred to as relative-movement time). This relative-movement equals the transit time of the ODL column with respect to a reference point in the OCT probe, such as a sensor comprised in the probe, the probe tip or a designated imaging location, if the probe is stationary. Said relative-movement time can then be used in some embodiments to derive microvascular parameters such as, but not limited to, absolute coronary flow, CFR, and microvascular resistance. In certain embodiments, the system may operate with controlled probe pullback speeds, additionally or instead of in stationary mode, enabling comparison of relative-movement times for different pullback speeds, which allows the processing unit to calculate the transit times of the ODL column as well as the column lengths. This comparison may also be performed with a pullback speed equal to zero (stationary mode) and a pullback speed different than zero (pullback mode). Additionally, the system is capable of functioning under both resting and hyperaemia conditions, allowing comprehensive physiological assessments.

By utilizing off-the-shelf OCT equipment, the present invention offers a cost-effective alternative to traditional methods, with the advantage that the practitioners are already familiarized with the setup. Its integration with existing catheterization tools simplifies workflows, reduces procedural complexity, and makes advanced coronary microvascular diagnostics more accessible to a broader range of healthcare providers. Furthermore, its ability to measure all relevant microvascular function parameters enable more precise diagnostics and better-informed clinical decision-making.

This invention represents a groundbreaking advancement in coronary microvascular assessment, addressing the limitations of current state-of-the-art techniques and offering a simpler, faster, and more versatile solution.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

In a first aspect of the invention, an imaging system, preferably an Optical Coherence Tomography (OCT) system, is disclosed. The imaging system of the invention comprises an imaging probe, preferably an OCT probe (1), and a processing unit in communication with the imaging probe, preferably with the OCT probe (1). It is understood that the imaging probe is suitable for or configured to be placed inside a blood vessel (3), preferably inside a coronary artery or microvasculature. The processing unit of the system of the invention is configured to follow a set of instructions stored in a processor-readable medium, preferably also comprised in the system of the invention, such as an OCT system, that when read by the processing unit or processor cause the processing unit to perform the following steps:
a) detect the time *t*₀ at which the distal end (21) of a first travelling column of a probe detectable liquid, preferably an OCT-detectable liquid (ODL) (2), of volume *V_{c}* travelling inside the blood vessel (3) towards the imaging probe tip at a velocity *v_{c}* reaches a reference point (11) in the imaging probe (1), and the time *t*₁ at which the proximal end (22) of the first travelling column of probe-detectable liquid reaches said reference point (11) in the imaging probe;
b) calculate a relative-movement time *T*₁ of the probe-detectable liquid column (2) with respect to the reference point (11) of the imaging probe (1) as a function of *t*₀ and *t*₁, preferably as the difference between *t*₁ and *t*₀; and
c) calculate one or more haemodynamic parameters associated to the blood vessel (3) as a function of *T*₁, preferably wherein said haemodynamic parameters are the absolute coronary flow, the coronary flow reserve and/or the microvascular resistance.

It is noted that the imaging system mentioned above is preferably an OCT system, and the probe-detectable liquid is preferably an OCT-detectable liquid (ODL).

It is noted that the OCT system operates using the principles of optical coherence tomography, a high-resolution imaging modality that utilizes low-coherence interferometry to capture detailed cross-sectional images of biological tissues. In some embodiments, the OCT probe (1) may include components such as fibre optics, light sources, detectors, and sensors optimized for imaging within blood vessels (3). The probe may have various shapes and sizes, such as cylindrical, tapered, or flexible configurations, to accommodate different vessel anatomies and insertion techniques. It is further noted that the OCT probe (1) may be designed for single-use or multiple uses, constructed from biocompatible materials such as polymers, stainless steel, or other metals with coatings to minimize friction and enhance biocompatibility.

It is also noted that the process of measuring the time *t*₀ and *t*₁ at which the distal end (21) and proximal end (22) of a travelling column of OCT-detectable liquid (2) reach a reference point (11) in the OCT probe (1) may be performed using a variety of sensing techniques. The reference point (11) may correspond, in some embodiments, to the OCT probe tip, a designated sensor, or an optical detection element. For instance, the arrival of the ODL column (2) at the reference point (11) may be detected through changes in refractive index, color, opacity, or backscatter intensity as captured by the imaging system. Alternatively, the reference point (11) may be defined by the position of a camera or a light-sensitive sensor within the probe (1), such as the camera or sensor themselves, where changes in the captured image indicate the presence of the ODL (2). It is also noted that the reference point (11) may comprise multiple detection regions or sensors along the length of the probe to enhance measurement precision.

The term relative-movement time *T*₁ is defined as the interval during which the column of ODL (2) interacts with, or passes over, or reaches, the reference point (11) of the OCT probe (1). In embodiments where the OCT probe (1) remains stationary within the blood vessel (3), *T*₁ corresponds directly to the transit time of the ODL column (2). In cases where the OCT probe (1) is in motion, such as in pullback mode, or pull-forward mode, depending if the probe moves backwards or forward, the relative-movement time may be adjusted based on the probe's velocity relative to the column (2), in order to obtain the transit time of the ODL column (2). It is noted that the relative-movement time can be calculated as the difference between *t*₁ and *t*₀, but may also be determined through other computational methods or algorithms, depending on the configuration of the system.

The haemodynamic parameters calculated by the system may comprise, but are not limited to, absolute coronary flow, coronary flow reserve (CFR), and/or microvascular resistance. In some embodiments, additional parameters such as flow velocity, coronary leakage, transit time, shear stress, or vascular compliance may also be derived. The system may employ automated algorithms to convert measured time intervals into velocities if the length of the ODL column (2) is known or to calculate the length of the column if time and velocity data are available. Thus, the scope invention covers all the alternatives regarding measuring one or two of these quantities and calculating the other. These computations may be performed in real-time to provide immediate feedback for clinical decision-making.

Advantageously, the described OCT system enables precise measurement of haemodynamic parameters using widely available OCT imaging technology. By integrating the detection of ODL transit with sophisticated processing capabilities, the system eliminates the need for specialized equipment such as Doppler wires or thermodilution catheters, thereby reducing cost and complexity, as well as the need to familiarize the practitioners with this new equipment. Instead, they can use a well know OCT system which is readily available. The possibility to obtain flow dynamic parameters, whether the probe is stationary or in motion, provides an enhancement in the utility of OCT systems and provides and invaluable and precise system and methodology for the assessment of coronary microvascular function.

It is noted that while the invention describes as a preferred embodiment the use of Optical Coherence Tomography (OCT) as the imaging technique, the scope of the invention is not limited to OCT. Other imaging modalities may also be employed, provided they can detect the presence, arrival, and departure of a liquid with optical properties different from blood. Examples of such imaging techniques include intravascular ultrasound (IVUS), near-infrared spectroscopy (NIRS), fluorescence imaging, or other optical-based methods capable of distinguishing a liquid within the blood vessel. In some embodiments, the system may include probes equipped with cameras or optical sensors designed to detect changes in optical properties, such as transparency, refractive index, or colour, enabling the detection of the travelling liquid column. These alternative systems allow for flexibility in implementation, enabling compatibility with different clinical setups and applications. For simplicity, hereinafter the system will be referred to an OCT system, but all embodiments could refer as well to an imaging system in a broader conception, encompassing other imaging alternatives, provided that the imaging system can detect the arrival and departure of a imaging system detectable liquid or probe detectable liquid.

The liquid used for detection may be referred to as a "probe-detectable liquid" (PDL), instead of ODL, to encompass a broad range of liquids that can be identified by the mentioned imaging or detection system alternative to OCT. Such liquids may include contrast agents, transparent fluids, fluorescent dyes, or any substance with optical or physical properties that differentiate it from blood. Examples of PDLs include saline solutions, iodinated contrast agents, fluorescent-tagged liquids, or any other biocompatible fluid that can be safely injected into blood vessels. In some embodiments, the PDL may have adjustable properties, such as viscosity or optical density, to optimize its detectability and compatibility with the chosen imaging system. The system may be configured to detect the arrival and departure of the PDL column by observing changes in the captured image, intensity, or optical scatter at the reference point. Additionally, the reference point may be calibrated to account for variations in the optical properties of the PDL, ensuring accurate and consistent detection across different liquid compositions.

Advantageously, the inclusion of alternative imaging techniques and the use of a broadly defined probe-detectable liquid (PDL) provide flexibility and expand the applicability of the invention to diverse clinical and research environments. This approach enables the system to be adapted to different imaging technologies and liquid types, ensuring compatibility with existing equipment while maintaining the ability to deliver precise haemodynamic assessments.

In a preferred embodiment of the invention, the OCT probe (1) is configured to remain stationary inside the blood vessel (3) during the travelling of the first ODL column (2), and wherein the processing unit is further configured to:
d) calculate the absolute coronary flow Q₁ as a function of *V_{c}* and the relative-movement time *T*₁.

It is noted that in some embodiments, the OCT probe (1) is configured to remain stationary inside the blood vessel (3) during the passage of the first travelling column of OCT-detectable liquid (ODL) (2). The stationary configuration eliminates the influence of probe movement on the measurements and allows for a straightforward calculation of some relevant parameters, given that the ODL volume *V_{c}* is known, or approximating it to the injected ODL volume, which is understood to be known. The stationary position may be achieved using various techniques, such as just leaving it to remain in place, anchoring the probe (1) within the vessel or employing a guiding catheter to stabilize its position. The probe (1) may include features such as friction-reducing coatings, balloon supports, or flexible materials to facilitate precise positioning while minimizing the risk of vessel trauma.

The processing unit is configured to calculate the absolute coronary flow Q₁ as a function of the volume *V_{c}* of the ODL column (2) and the relative-movement time *T*₁. In this stationary configuration, *T*₁ corresponds directly to the transit time of the ODL column (2) passing the reference point (11) of the OCT probe (1). The calculation of Q₁ may involve dividing *V_{c} by T*₁, providing a straightforward relationship between the known volume of the liquid column and the time it takes to traverse the reference point. The volume *V_{c}* may be predetermined, for instance, by an automated injector, a syringe with volumetric indicators, or calculated based on the physical dimensions of the column as detected by the OCT imaging system.

It is further noted that in some embodiments, the system may automatically adjust for variations in ODL properties, such as viscosity or temperature, to enhance the accuracy of the flow calculation. Alternative methods for calculating Q₁ may include using flow models or algorithms that account for vessel geometry, the rheological properties of the ODL, or physiological conditions such as blood pressure and vessel elasticity. The system may also provide real-**time visualization** of the ODL column (2) to confirm proper injection and detection, ensuring the reliability of the measurement.

Advantageously, a stationary configuration of the OCT probe (1) simplifies the measurement process by eliminating the influence of probe movement, enabling precise and reliable calculations of absolute coronary flow. By using the relationship between *V_{c}* and *T*₁, the system provides a direct and efficient method for determining coronary flow, reducing procedural complexity and improving the utility of OCT-based diagnostics in clinical settings.

In a preferred embodiment of the invention, wherein the processing unit of the system is further configured to:
e) perform previously mentioned steps (a) and (b), i.e., relative-movement time determination, in hyperaemia conditions of the vessel (3) to calculate a hyperaemia relative-movement time *T*_{1*H*} of the ODL column (2) with respect to the OCT probe (1); and
f) estimate a coronary flow reserve (CFR) parameter as a function of *T*₁ and *T*₁*_{H},* preferably as the ratio between *T*_{1*H*} and *T*₁

In some embodiments, the OCT system is further configured to perform the steps to calculate the relative-movement time of the ODL column (2) with respect to the OCT probe (1) in hyperaemia conditions of the blood vessel (3). Hyperaemia conditions refer to a physiological state of increased blood flow through the vessel, for example induced pharmacologically using agents such as adenosine, papaverine, or nitro-glycerine. These agents dilate the coronary vessels and minimize microvascular resistance, enabling the measurement of maximal flow conditions.

The processing unit may therefore be configured to calculate a hyperaemia relative-movement time *T*₁*_{H},* which represents the time taken for the column of OCT-detectable liquid (ODL) (2) to traverse the reference point (11) of the OCT probe (1) under hyperaemia conditions. Similarly to *T*₁, *T*_{1*H*} may be derived from the time intervals *t*₀ and *t*₁ corresponding to the arrival and departure of the proximal and distal ends of the ODL column (2) at the reference point (11). In some embodiments, the system may adjust the calculation of *T*_{1*H*} to account for any changes in vessel geometry or flow dynamics specific to the hyperaemia state.

The system is preferably further configured to estimate the coronary flow reserve (CFR) parameter as a function of *T*₁ and *T*₁*_{H}.* In some embodiments, CFR is calculated as the ratio between *T*_{1*H*} and *T*₁, or *T*₁/*T*₁*_{H},* providing a direct and intuitive relationship between flow at maximal hyperaemia and baseline conditions. Alternatively, the system may employ advanced computational algorithms to derive CFR, incorporating additional factors such as vessel cross-sectional area, ODL properties, or flow dynamics, to enhance accuracy and versatility.

It is further noted that CFR represents a critical parameter for assessing the functional capacity of the coronary circulation. In some embodiments, the system may display or record the CFR value in real-time, allowing clinicians to assess coronary health during the procedure. The processing unit may also provide additional insights, such as identifying pathological thresholds for CFR indicative of coronary microvascular dysfunction or ischemia.

Advantageously, the ability to calculate *T*_{1*H*} and estimate CFR in hyperaemia conditions enables the OCT system to provide a more complete evaluation of coronary microvascular function. The system thus offers a robust and efficient method for diagnosing CMD and assessing coronary reserve capacity, while integrating seamlessly with standard catheterization workflows. This ensures precise, reproducible, and clinically relevant measurements without requiring additional specialized equipment.

According to a preferred embodiment of the invention, the probe is further configured to be displaced at a pullback speed *PBs*₁, preferably when the first column of ODL (2) is travelling inside the vessel (3). It is understood that this pullback speed can be configured and comprises a wide range of values or interval of values, for instance the probe may be configured to be displaced as well at a pullback speed *PBs*₂*,* different than *PBs*₁, preferably when a second column of ODL (2) of volume *V_{c}* is travelling inside the vessel (3) towards the OCT probe tip at a velocity *v_{c}.* For example, *PBs*₂ may be 1 cm/s and *PBs*₁ 3 cm/s, or *PBs*₂ may be 0 cm/s (remain stationary) and *PBs*₁ 5 cm/s, etcetera. It is noted that in order to calculate the velocities of the columns of ODL (2), preferably the first and second columns of ODL (2) are injected in such a way that both have the same volume *V_{c}* and velocity *v_{c}*, or approximately the same, preferably with a difference in volume and/or velocity less than 10%, more preferably less than 5%, even more preferably less than 2%, even more preferably less than 1%, even more preferably less than 0.3%. In some embodiments, the processing unit is further configured to perform the following steps:
e) optionally, calculate a first probe displacement length ${l}_{1}^{′}$ as a function of *T*₁ and *PBs*₁;
f) detecting the time ${t}_{0}^{′}$ at which the distal end (21) of the second travelling column of ODL (2) reaches a reference point (11) in the OCT probe (1), and the time ${t}_{1}^{′}$ at which the proximal end (22) of the travelling column of ODL (2) reaches said reference point (11) in the OCT;
g) calculate a relative-movement time *T*₂ of said second column of ODL (2) with respect to the OCT probe (1) reference point (11) as a function of ${t}_{0}^{′}$ and ${t}_{1}^{′}$, and optionally calculate a second probe displacement length ${l}_{2}^{′}$ as a function of *T*₂ and *PBs*₂;
h) calculate the ODL column (2) velocity *v_{c}* and/or the column length *l_{c},* as a function of *T*₁, *T*₂*, PBs*₁ and *PBs₂,* or as a function of ${l}_{1}^{′} , {l}_{2}^{′}$*, PBs*₁ and *PBs₂,* or as a function of *T*₁, ${l}_{2}^{′}$*, PBs*₁ and *PBs₂,* or as a function of ${l}_{1}^{′}$, *T₂, PBs*₁ and *PBs*₂;
i) preferably, calculate one or more haemodynamic parameters associated to the blood vessel (3) as a function of *v_{c}* and/or *l_{c}*, preferably wherein said haemodynamic parameters are the absolute coronary flow, the coronary flow reserve and/or the microvascular resistance

It is noted that in some embodiments the OCT probe (1) is configured to be displaced within the blood vessel (3) at controlled pullback speeds *PBs,* such as *PBs*₁ and *PBs₂* during the transit of two different columns of OCT-detectable liquid (2). The first pullback speed *PBs*₁ is applied to the OCT probe (1) during the movement of the first ODL column (2), and preferably a second pullback speed *PBs₂,* different from *PBs*₁, is applied during the movement of a second ODL column (2). In some embodiments, one of these speeds may be zero, such that the probe (1) remains stationary during one measurement, while the second measurement is at pullback speeds different than zero. Alternatively, both measurements may involve non-zero pullback speeds, ensuring versatility in system operation.

In some embodiments the processing unit is configured to calculate a first probe displacement length ${l}_{1}^{′}$, as a function of *T*₁ (the relative-movement time of the first ODL column (2)) and *PBs*₁ . Similarly, the processing unit may calculate a second probe displacement length ${l}_{2}^{′} ′$ as a function of *T*₂ (the relative-movement time of the second ODL column (2)) and *PBs₂.* The relative-movement time *T*₁ for the first ODL column (2) is determined by detecting the time *t*₀ at which the distal end (21) of the column reaches the reference point (11) of the OCT probe (1), and *t*₁ is the time at which the proximal end (22) of the ODL column (2) passes the reference point (11), wherein *T*₁ is equal to *t*₁ if *t*₀ = 0. Similarly, the relative-movement time *T*₂ for the second ODL column (2) is determined by detecting the time ${t}_{0}^{′}$ at which the distal end (21) of the column reaches the reference point (11) of the OCT probe (1), and ${t}_{1}^{′}$ is the time at which the proximal end (22) passes the reference point (11), wherein *T*₂ is equal to ${t}_{1}^{′}$ if ${t}_{0}^{′} = 0$. By using these time intervals and the known pullback speeds, the system can precisely derive the length and velocity of the ODL column (2).

**The velocity** *v_{c}* of the ODL column (2) can be calculated as a function of the relative-movement times (*T*₁ and *T*₂) and the pullback speeds (*PBs₁* and *PBs*₂), or, alternatively, as a function of the probe displacement lengths ( ${l}_{1}^{′}$ and ${l}_{2}^{′}$) and the pullback speeds (*PBs*₁ and *PBs*₂)*.* Measuring *T*₁ and *T*₂ in two separate measurements provides two equations that resolve two unknowns: the velocity *v_{c}* of the ODL column (2) and its length *l_{c}.* The system assumes that the first and second ODL columns (2) have the same or approximately the same velocity and length, with an allowable error margin of, for example, ±1%, ±5% or ±10%. It is understood that more measurements for a third, fourth, etc ODL columns may be performed for increased precision. It is also understood that the OCT probe (2) pullback speeds are always known, as they are set before starting the measurement.

In some embodiments, the movement of the OCT probe (1) is referred to as pullback, if it moves in the opposite direction to the velocity *v_{c}* of the ODL column (2). Alternatively, the system may operate in forward mode, where the probe moves in the same direction as the ODL column (2), though this configuration is less preferred due to the smaller velocity differentials, increased complexity, and longer displacement distances required for accurate measurement. In forward mode, it is noted that the probe velocity must be different from the column velocity.

Haemodynamic parameters associated with the blood vessel (3), such as absolute coronary flow, coronary flow reserve (CFR), and microvascular resistance, can be calculated as a function of the derived ODL column velocity *v_{c}* and/or column length *l_{c}.*

Advantageously, the configuration to measure relative-displacement lengths two times at different pullback speeds, allows the OCT system to resolve the length and velocity of the ODL column (2) with high precision, ensuring accurate characterization of vessel dynamics. By enabling the precise calculation of haemodynamic parameters, such as, but not limited to, absolute flow, CFR, and microvascular resistance, this system offers a robust and efficient solution for assessing coronary and microvascular function, obtaining the actual values of the haemodynamic parameters in an analytical way, and, also importantly, integrating seamlessly into existing clinical workflows.

Figure 2 shows a schematic representation of a column of OCT-Detectable liquid (2) travelling inside a blood vessel (3) in the forward direction, whereas an OCT probe (1) is being displaced inside said blood vessel (3) in the opposite direction, at a pullback speed *PBs.* The length of the ODL column is *l_{c},* and, as it can be seen in Figure 1(a), at time *t*₀ the distal end (21) of the ODL column (2) is in contact with or at the same level as the reference point (11) of the OCT probe (1), in this case the distal part of the tip, but in some other embodiments it could be any other reference point (11) such as a sensor or a detector themselves or their position in the probe. At time *t*₁, a relative-movement time *T*₁ has passed, wherein *T*₁ = *t*₁ *- t*₀*.* As it can be observed in Figure 1(b), at time *t*₁ the proximal end (22) of the ODL column (2) is in contact with or at the same level as the reference point (11). At that moment *(i. e., t*₁), the ODL column (2) finds itself to be displaced one ODL column length *l_{c}* with respect to the reference point (11) of the OCT probe (1). Since the ODL column (2) is moving forward, and the OCT probe (1) is moving backwards, the relative displacement is the sum of the absolute value of the distance displacement of the ODL column (2), ${l}_{1}^{"}$, and the absolute value of the distance displacement of the OCT probe, ${l}_{1}^{′}$*,* with respect to a stationary point such as a point in the blood vessel, or the blood vessel itself taken as displacement axis. Therefore, according to Figure 1(b), at t = *t*₁ the length of the column is equal to the sum of the displacements, in absolute value, of the ODL column (2) and the OCT probe (1), ${l}_{c} = {l}_{1}^{′} + {l}_{1}^{"}$*.*

It is noted that sometimes the length of the column and its velocity are unknown, in which case, even if the time is measured, it is not possible to find their exact value only with one measurement because the equation of movement would have two unknowns. In these cases, the exact length and velocity of the ODL column (2) can be obtained if this measurement or group of steps is performed for two ODL columns (2) of the same velocity *v_{c}* and length *l_{c},* but with different pullback speeds of the OCT probe (1), according to the following equation: ${v}_{c} = \frac{{l}_{2}^{′} - {l}_{1}^{′}}{\frac{{l}_{1}^{′}}{{PBs}_{1}} - \frac{{l}_{2}^{′}}{{PBs}_{2}}}$

**Wherein** ${l}_{1}^{′}$ and ${l}_{2}^{′}$ is the distance that the OCT probe (2) has been displaced in pullback mode, in absolute value, in the first and second measurements respectively, and *PBs*₁ and *PBs₂* is the pullback speed of the OCT probe (2) in the first and second masurements, respectively, and in absolute value, for a first and a second column of ODL (2), wherein said first and second ODL columns have the same or approximately the same velocity *v_{c}* and length *l_{c}.*

An equivalent calculation of the ODL column (2) velocity *v_{c}*, but employing the measured relative-movement times *T*₁ and *T*₂ of the first and second columns (2), respectively, and their respective pullback speeds *PBs*₁ and *PBs₂* is: ${v}_{c} = \frac{\left|{PBs}_{2}\right| ⋅ {T}_{2} - \left|{PBs}_{1}\right| ⋅ {T}_{1}}{{T}_{1} - {T}_{2}}$

The equivalence of these formulas for *v_{c}* is expected from the relationship of pullback speed with displacement length of the OCT probe (1) and relative-movement time: ${PBs}_{1} = \frac{{l}_{1}^{′}}{{T}_{1}}$ and ${PBs}_{2} = \frac{{l}_{2}^{′}}{{T}_{2}}$

It is also noted that column velocity and length are correlated by the equation *v_{c} = l_{c}*/*TT,* wherein *TT* is the transit time of the ODL column (2). Additionally, the length may be calculated as: ${l}_{c} = \left|{v}_{c}\right| ⋅ T + \left|PBs\right| ⋅ T$

Which applies for both the first and second column (wherein *T* and *PBs* would be *T*₁ and *PBs*₁, and *T*₂ and *PBs₂* respectively).

Then, the transit time in pullback mode can be calculated as: $TT = \frac{{l}_{c}}{\left|{v}_{c}\right|} = T ⋅ \left(1+\frac{\left|PBs\right|}{\left|{v}_{c}\right|}\right)$

Therefore, calculating one or more haemodynamic parameters associated to the blood vessel (3) as a function of *v_{c}* and/or *l_{c},* and relative-movement time *T* is straightforward once these parameters are known.

### Absolute Coronary Flow (ACF)

${Φ}_{c} = \frac{{V}_{c}}{TT}$

Wherein *V_{c}* is the volume of the ODL travelling column (2), preferably approximated as the injected volume of ODL, if the volume is known and spillover into other blood vessels such as the aorta can be neglected. This injected volume thus can generally be controlled before injection by setting it up in an automatic injector or by measuring it with a syringe volumetric indicator if manually injected with said syringe. For example, if an automatic injector is set to inject 3 ml per second during one second, then *V_{c}* is equal to, or can be approximated to, 3 ml. On the other hand, *TT,* is the transit time of the travelling ODL column (2).

### Coronary Flow Reserve (CFR)

$CFR = \frac{{v}_{cH}}{{v}_{c}}$

Wherein *v_{cH}* is the ODL column (2) velocity in hyperaemia conditions, and *v_{c}* is the ODL column (2) velocity in normal or resting conditions.

Additionally, the CFR may be also calculated as a relationship of the transit time in basal or normal conditions (*TT_{c}*) and the transit time in hyperaemia conditions (*TT_{cH}*), as: $CFR = \frac{{TT}_{c}}{{TT}_{cH}}$

Alternatively, the CFR may be calculated by employing the absolute coronary flow at hyperaemia conditions (Φ*_{cH}*) and at normal conditions (Φ*_{c}*) as: $CFR = \frac{{Φ}_{cH}}{{Φ}_{c}}$

### Microvascular resistance

${R}_{μ} = \frac{Δ P}{{Φ}_{c}}$

Wherein Δ*P* is the pressure difference at the venous end of the capillary (*Pᵥ*) with respect to the distal pressure at the arterial side (*P_{d}*). It is noted that Δ*P* can be calculated by means of computational physiology, preferably by optical flow ratio (OFR), also, generally *Pᵥ* is negligible compared to *P_{d}*, and therefore Δ*P* may be approximated as Δ*P = P_{d}.*

In a preferred embodiment of the invention, the processing unit is further configured to perform the following steps: calculate the ODL column (2) velocity in hyperaemia conditions *v_{cH}*; and calculate the coronary flow reserve CFR as a function of *v_{cH}* and *v_{c}*, preferably as the ratio between *v_{cH}* and *v_{c}.*

In some embodiments, the system may use additional computational methods or correction factors to account for variations in vessel geometry, ODL properties, or physiological parameters to enhance the accuracy and reliability of the CFR calculation.

It is further noted that CFR is an important parameter in assessing the functional capacity of the coronary microvasculature. A higher CFR indicates a healthy ability of the coronary circulation to increase flow in response to demand, while a reduced CFR may suggest microvascular dysfunction or ischemia. In some embodiments, the system may include diagnostic thresholds or alerts to identify pathological CFR values, streamlining clinical decision-making.

Advantageously, the system of the invention enables the direct measurement of CFR. The disclosed OCT system, thus, provides a comprehensive and efficient method for evaluating coronary microvascular function. The integration of hyperaemia-based measurements enables the precise obtention of CFR values, providing a robust, accurate, and clinically relevant assessment of coronary physiology.

In a preferred embodiment of the invention, the system of the invention is further configured to calculate the absolute coronary flows *Q*₁ and/or *Q*₂ of the first and second ODL columns (2) respectively, as a function of the volume *V_{c}* and the transit time *TT*₁ and/or *TT*₂ of the first and second ODL columns respectively, wherein the transit time of the first column, *TT*₁, is calculated as a function of *PBs*₁, *v_{c}* and *T*₁, and the transit time of the second column, *TT*₂, is calculated as a function of *PBs*₂*, v_{c}* and *T*₂. It is noted that the volume *V_{c}* is the same or approximately the same for the first and second columns. It is also noted that *T*₁ and *T*₂ are the relative-movement time of the first and second column, respectively, with respect to the reference point of the probe. If the probe is being displaced in an opposite direction than the movement of the column, then said relative-movement time will be shorter than the transit time. In other words, the relative-movement time is the same as the transit time when the probe is stationary and only the column is moving.

It is also noted that the absolute coronary flows *Q*₁ and/or *Q*₂ provide important insights into coronary circulation. In some embodiments, the system may display these values in real time or record them for later analysis.

In alternative embodiments, the system may use calibration techniques to enhance the precision of flow measurements. For example, the processing unit may adjust the value of *V_{c}* or *v_{c}* based on environmental conditions, such as temperature or pressure, to provide increased consistency across different patients or procedures. Furthermore, the calculations may incorporate error correction algorithms to compensate for potential discrepancies arising from variations in ODL injection rates or non-uniform flow conditions, or differences in the length, volumes and velocities of the ODL columns injected.

Advantageously, this embodiment allows for the precise calculation of absolute coronary flows using transit times derived from the system and method of the invention. By employing measurements of pullback speeds and relative-movement times, the system provides accurate and reliable flow quantifications. This capability greatly improves the diagnostic power of the OCT system, enabling comprehensive assessment of coronary dynamics and microvascular function while maintaining flexibility for diverse procedural conditions.

In a preferred embodiment of the invention, the processing unit of the system is further configured to: image with the OCT probe (1) the blood vessel (3) and estimate the pressure difference Δ*P* at the venous end of the capillary (*Pᵥ*) with respect to the distal pressure at the arterial side (*P_{d}*), or alternatively, if *Pᵥ* is negligible with respect to *P_{d}*, then calculate instead the pressure P_{d} at a distal point of the vessel (3) by means of computational physiology, preferably by optical flow ratio (OFR); and calculate a microvascular resistance parameter Rₘ as a function of the pressure P_{d} and the absolute coronary flow *Q*₁ or *Q*₂, or as a function of the pressure P_{d} and the transit time *TT*₁ or *TT*₂*.*

The microvascular resistance *Rₘ* represents an important physiological parameter that directly reflects the functional state of the coronary microvasculature. In some embodiments, Rₘ may be calculated using Ohm's law analogues for blood flow, where resistance is proportional to the pressure gradient divided by the flow.

It is further noted that the OCT probe (1) may acquire high-resolution images of the vessel to support the computational estimation of *P_{d}*, and the calculation of *Rₘ*. Such imaging may be used in some embodiments to delineate vessel morphology, identify structural abnormalities, or refine flow models. In some embodiments, additional data such as vessel cross-sectional area or ODL column velocity may be integrated into the calculations to improve accuracy and reliability.

In some additional embodiments, the system may employ correction factors or calibration techniques to account for variations in vessel conditions, such as irregular geometries, stenosis, or branching patterns. Furthermore, the processing unit may incorporate machine learning algorithms or predictive models to enhance the precision of *P_{d}* estimation and *Rₘ* calculation.

Advantageously, the system of the invention allows for the precise calculation of microvascular resistance *Rₘ* using imaging and flow data derived from the OCT probe (1). By integrating computational physiology techniques such as OFR, the system provides a non-invasive and efficient means to assess further coronary microvascular function parameters.

In a preferred embodiment of the invention, the system of the invention further comprises, or is further configured to be connected to, an automatic injector of ODL, able to infuse a column of known volume *V_{c}* of ODL at a constant flow rate (*Qᵣ*) during a predetermined infusion time (*Tᵢ*).

The automatic injector may be integrated into the OCT system or operate as an independent unit that communicates with the processing unit. In some embodiments, the injector may include advanced control systems to regulate the infusion parameters in real-time, compensating for variations in vessel conditions or procedural requirements. The injector may also provide feedback to the processing unit, allowing synchronization between the infusion process and the imaging or data acquisition by the OCT probe (1).

It is further noted that the volume injected may be selected based on the dimensions and flow dynamics of the targeted blood vessel (3). In some embodiments, the volume may range from small amounts for microvascular studies to larger volumes for imaging of larger vessels. The flow rate *Qᵣ* in some embodiments may be set to provide optimal displacement of blood within the vessel, avoiding turbulence or incomplete displacement. Typical flow rates may range from 0.1 mL/s to 10 mL/s, preferably between 1 mL/s and 5 mL/s, depending on the vessel size and the properties of the ODL.

The ODL infused by the automatic injector may include biocompatible liquids such as contrast agents, saline solutions, or other liquids with optical properties distinct from blood. In some embodiments, the injector may include temperature regulation or mixing capabilities to maintain the ODL at optimal conditions for infusion and imaging.

In alternative embodiments, the injector may support programmable infusion patterns, allowing for dynamic control over the infusion process. For example, the system may perform multiple infusions of varying volumes, flows or infusion times to enhance data collection and analysis. The injector may also be equipped with safety features, such as flow sensors or pressure monitors, to prevent over-infusion or adverse events during the procedure.

Advantageously, the inclusion or connectivity to an automatic injector allows the OCT system to deliver consistent and controlled ODL columns, enhancing the accuracy and reproducibility of haemodynamic measurements. By standardizing the infusion process, the system minimizes operator variability, optimizes imaging conditions, and supports a wide range of clinical and research applications, improving the overall reliability and utility of the invention.

In a preferred embodiment of the invention, the system further comprises a guide-extension catheter to inject the ODL column (2).

It is noted that in some embodiments the guide-extension catheter is designed to extend the reach of the ODL infusion system, allowing precise delivery of the liquid into the targeted blood vessel (3). This component enhances allows for injection of the ODL column (2) at specific anatomical locations, such as distal to a major vessel branch or within microvascular regions. The guide-extension catheter may comprise a flexible, biocompatible tubular structure designed to navigate through the vasculature with minimal risk of damage to the vessel walls. In some embodiments, the catheter may include radiopaque markers or embedded sensors to assist with real-time imaging and navigation during placement. The catheter's internal lumen is preferably configured to accommodate the passage of the ODL column (2) while maintaining its integrity and ensuring accurate flow dynamics during infusion.

It is further noted that the guide-extension catheter may be compatible with various injector systems, allowing seamless integration with automatic injectors or manual infusion setups. In some embodiments, the catheter may be equipped with features to minimize ODL leakage or backflow, such as a valve mechanism at its distal end or a sealing system that maintains a constant flow rate. The catheter may also include in some embodiments multiple side ports to ensure uniform distribution of the ODL within the vessel, which may be particularly advantageous in complex anatomical regions. In some embodiments, the guide-extension catheter may be designed for compatibility with a range of ODL compositions, including viscous contrast agents, saline solutions, or other biocompatible liquids. The catheter may also support interchangeable tips or adapters to accommodate vessels of varying sizes or to optimize infusion for specific clinical scenarios. For instance, narrower tips may be employed for microvascular applications, while larger-diameter tips may be used for larger coronary arteries or peripheral vessels.

The use of a guide-extension catheter enables precise placement of the infusion point, which is particularly important in scenarios where the ODL column (2) must displace blood within a localized segment of the vessel without significant dispersion or mixing. This ensures optimal conditions for OCT imaging and subsequent haemodynamic calculations.

Advantageously, the inclusion of a guide-extension catheter provides enhanced control over the delivery of the ODL column (2), allowing for accurate and localized infusion in a wide variety of vessel types and anatomical locations. For example, it allows to know precisely the volume of the column or to confidently approximate it to the volume infused, given that the infusion point is close to the reference point of the OCT probe. This configuration allows to improve the precision of haemodynamic measurements and expands the applicability of the OCT system to more complex clinical and research environments, while maintaining safety and procedural efficiency.

In a second aspect of the invention, a computer program is provided. The computer program comprising instructions which, when executed by a processor, causes the processor to carry out the following steps:
(I) detecting the time *t*₀ at which the distal end (21) of a first travelling column of ODL (2) of known volume *V_{c}* travelling at a velocity *v_{c}* towards the tip of an OCT probe (1) placed inside a blood vessel (3), reaches a reference point (11) in the OCT probe (1), and the time *t*₁ at which the proximal end (22) of the first travelling column of ODL (2) reaches said reference point (11) in the OCT;
(II) calculating a relative-movement time *T*₁ of the ODL column (2) with respect to the reference point (11) of the OCT probe (1) as a function of *t*₀ and *t*₁, preferably as the difference between *t*₁ and *t*₀;
(III) calculating one or more haemodynamic parameters associated to the blood vessel (3) as a function of *T*₁, preferably wherein said haemodynamic parameters comprise the absolute coronary flow, the coronary flow reserve and/or the microvascular resistance, more preferably the haemodynamic parameters comprise the absolute coronary flow Q₁ calculated as a function of *V_{c}* and the relative-movement time *T*₁.

In a preferred embodiment of the computer program of the invention, the computer program when executed by a processor, causes the processor to further carry out the following steps:
(IV) estimating a coronary flow reserve (CFR) parameter as a function of *T*₁ and *T*₁*_{H},* preferably as the ratio between *T*_{1*H*} and *T*₁, wherein *T*_{1*H*} is a hyperaemia relative-movement time *T*_{1*H*} of the ODL column (2) with respect to the reference point (11) of the OCT probe (1), obtained performing steps (i) to (iv) in hyperaemia conditions of the vessel (3).

In a preferred embodiment of the computer program of the invention, the computer program when executed by a processor, causes the processor to carry out the following steps:
(I) detecting the times *t*₀ and ${t}_{0}^{′}$, respectively, at which the distal end (21) of the first and a second columns of ODL (2) both having each a volume *V_{c}* and both travelling inside a blood vessel (3) at a velocity *v_{c}*, reach the reference point (11) in the OCT probe (1), and the times *t*₁ and ${t}_{1}^{′}$ at which the proximal end (22) of the first and second travelling columns of ODL (2), respectively, reach said reference point (11) in the OCT, wherein the OCT probe (1) is moving at a pullback speed *PBs*₁ with respect to the first column of ODL (2) and at a pullback speed *PBs*₂ with respect to the second column of ODL (2);
(II) calculating relative-movement times *T*₁ and *T*₂ of the first and second ODL columns (2), respectively, with respect to the reference point (11) of the OCT probe (1) as a function of *t*₀ and *t*₁, and as a function of ${t}_{0}^{′}$ and ${t}_{1}^{′}$, respectively, preferably as the difference between *t*₁ and *t*₀ and between ${t}_{1}^{′}$ and ${t}_{0}^{′}$;
(III) optionally, calculating a first probe displacement length ${l}_{1}^{′}$ as a function of *T*₁ and *PBs*₁*,* and a second probe displacement length ${l}_{2}^{′}$ as a function of *T*₂ and *PBs₂*;
**(IV) calculating** the ODL column (2) velocity *v_{c}* as a function of *T*₁, *T*₂*, PBs*₁ and *PBs₂,* or as a function of ${l}_{1}^{′} , {l}_{2}^{′}$, *PBs*₁ and *PBs₂,* wherein the ODL column (2) velocity *v_{c}* is related to the blood flow rate.

It is noted that in the computer program and in the method of the invention, both columns of ODL (2) are preferably not infused into the blood vessel simultaneously, but one after the other, more preferably the second ODL column is infused after the first ODL column has passed over the OCT probe (1) and after said OCT probe has already been displaced in pullback mode for a first time. That is, preferably the measurements are taken sequentially.

In a preferred embodiment of the computer program of the invention, the computer program when executed by a processor, causes the processor to further carry out the following steps:
(I) calculating the ODL column (2) velocity in hyperaemia conditions *v_{cH}*;
(II) calculating the coronary flow reserve CFR as a function of *v_{cH}* and *v_{c}*, preferably as the ratio between *v_{cH}* and *v_{c}.*

In a preferred embodiment of the computer program of the invention, the computer program when executed by a processor, causes the processor to further carry out the following steps:
(I) calculating the absolute coronary flows *Q*₁ and/or *Q*₂ as a function of *V_{c}* and the transit time *TT*₁ of the first ODL column (2) for *Q*₁ or the transit time *TT*₂ of the second ODL column (2) for *Q*₂, wherein *TT*₁ is calculated as a function of *PBs*₁, *v_{c}* and *T*₁, and *TT*₂ is calculated as a function of *PBs*₂*, v_{c}* and *T*₂.

In a preferred embodiment of the computer program of the invention, the computer program when executed by a processor, causes the processor to further carry out the following steps:
(I) imaging with the OCT probe (1) the blood vessel (3) and estimate the pressure P_{d} at a distal point of the vessel (3) by means of computational physiology, preferably by optical flow ratio (OFR); and
calculating a microvascular resistance parameter Rₘ as a function of the pressure P_{d} and the absolute coronary flow *Q*₁ or *Q*₂, or as a function of the pressure P_{d} and the transit time *TT*₁ or *TT*₂.

In a third aspect of the invention, a methodology to calculate one or more haemodynamic parameters associated to a blood vessel (3) is provided, preferably wherein said haemodynamic parameters are the absolute coronary flow, the coronary flow reserve and/or the microvascular resistance. The method of the third aspect of the invention comprises the following steps:
(I) placing an OCT probe (1) inside the blood vessel (3),
(II) injecting a first ODL column (2) of known volume *V_{c}* inside the blood vessel (3) in such a way that it travels towards the OCT probe tip at a velocity *v_{c}*,
(III) detecting the time *t*₀ at which the distal end (21) of a first travelling column of ODL (2) of known volume *V_{c}* travelling at a velocity *v_{c}* towards the tip of an OCT probe (1) placed inside a blood vessel (3), reaches a reference point (11) in the OCT probe (1), and the time *t*₁ at which the proximal end (22) of the first travelling column of ODL (2) reaches said reference point (11) in the OCT;
(IV) calculating a relative-movement time *T*₁ of the ODL column (2) with respect to the reference point (11) of the OCT probe (1) as a function of *t*₀ and *t*₁, preferably as the difference between *t*₁ and *t*₀;
(V) calculating one or more haemodynamic parameters associated to the blood vessel (3) as a function of *T*₁, preferably wherein said haemodynamic parameters are the absolute coronary flow, the coronary flow reserve and/or the microvascular resistance, more preferably the haemodynamic parameters comprise the absolute coronary flow Q₁ calculated as a function of *V_{c}* and the relative-movement time *T*₁.

In a preferred embodiment of the third aspect of the invention, the method further comprises the step of:
(VI) estimating a coronary flow reserve (CFR) parameter as a function of *T*₁ and *T*_{1*H*}, preferably as the ratio between *T*_{1*H*} and *T*₁, wherein *T*_{1*H*} is a hyperaemia relative-movement time *T*_{1*H*} of the ODL column (2) with respect to the reference point (11) of the OCT probe (1), obtained performing steps (i) to (iv) in hyperaemia conditions of the vessel (3).

In a preferred embodiment of the third aspect of the invention, the method further comprises the following steps:
(VII) detecting the times *t*₀ and ${t}_{0}^{′}$, respectively, at which the distal end (21) of the first and a second columns of ODL (2) both having each a volume *V_{c}* and both travelling inside a blood vessel (3) at a velocity *v_{c}*, reach the reference point (11) in the OCT probe (1), and the times *t*₁ and ${t}_{1}^{′}$ at which the proximal end (22) of the first and second travelling columns of ODL (2), respectively, reach said reference point (11) in the OCT, wherein the OCT probe (1) is moving at a pullback speed *PBs*₁ with respect to the first column of ODL (2) and at a pullback speed *PBs*₂ with respect to the second column of ODL (2);
(VIII) calculating relative-movement times *T*₁ and *T*₂ of the first and second ODL columns (2), respectively, with respect to the reference point (11) of the OCT probe (1) as a function of *t*₀ and *t*₁, and as a function of ${t}_{0}^{′}$ and ${t}_{1}^{′}$, respectively, preferably as the difference between *t*₁ and *t*₀ and between ${t}_{1}^{′}$ and ${t}_{0}^{′}$;
(IX) optionally, calculating a first probe displacement length ${l}_{1}^{′}$ as a function of *T*₁ and *PBs*₁, and a second probe displacement length ${l}_{2}^{′}$ as a function of *T*₂ and *PBs₂*;
(X) calculating the ODL column (2) velocity *v_{c}* as a function of *T*₁, *T₂*, *PBs*₁ and *PBs₂,* or as a function of ${l}_{1}^{′} , {l}_{2}^{′}$*, PBs*₁ and *PBs₂,* wherein the ODL column (2) velocity *v_{c}* is related to the blood flow rate.

It is noted that in the computer program and in the method of the invention, both columns of ODL (2) are preferably not infused into the blood vessel simultaneously, but one after the other, more preferably the second ODL column is infused after the first ODL column has passed over the OCT probe (1) and after said OCT probe has already been displaced in pullback mode for a first time. That is, preferably the measurements are taken sequentially.

In a preferred embodiment of the third aspect of the invention, the method further comprises the following steps:
(XI) calculating the ODL column (2) velocity in hyperaemia conditions *v_{cH}*;
(XII) calculating the coronary flow reserve CFR as a function of *v_{cH}* and *v_{c}*, preferably as the ratio between *v_{cH}* and *v_{c}.*

In a preferred embodiment of the third aspect of the invention, the method further comprises the following steps:
(XIII) calculating the absolute coronary flows *Q*₁ and/or *Q*₂ as a function of *V_{c}* and the transit time *TT*₁ of the first ODL column (2) for *Q*₁ or the transit time *TT*₂ of the second ODL column (2) for *Q*₂, wherein *TT*₁ is calculated as a function of *PBs*₁, *v_{c}* and *T*₁, and *TT*₂ is calculated as a function of *PBs*₂*, v_{c}* and *T*₂.

In a preferred embodiment of the third aspect of the invention, the method further comprises the following steps:
(XIV) imaging with the OCT probe (1) the blood vessel (3) and estimate the pressure P_{d} at a distal point of the vessel (3) by means of computational physiology, preferably by optical flow ratio (OFR); and
(XV) calculating a microvascular resistance parameter Rₘ as a function of the pressure P_{d} and the absolute coronary flow *Q*₁ or *Q*₂, or as a function of the pressure P_{d} and the transit time *TT*₁ or *TT*₂*.*

In a fourth aspect of the invention, an OCT system is used to determine coronary physiologic parameters according to any one of the embodiments of the method of the invention, preferably the OCT system is used to determine CFR, IMR, Qc, Rm, FFR and/or OFR, more preferably to determine CFR, IMR, Qc and/or Rm.

In a fifth aspect of the invention, a computer readable storage medium is disclosed comprising instructions which, when executed by a processor, cause the computer to determine coronary physiologic parameters according to any one of the embodiments of the method of the invention.

In a sixth aspect of the invention, a computer readable storage medium is disclosed comprising instructions which, when executed by a processor, cause the computer to carry out the steps comprised in any one of the embodiments of the computer program of the second aspect of the invention, or the computer readable storage medium comprises any one of the embodiments of the computer program of the second aspect of the invention.

To enable a better understanding of the present disclosure, reference will now be made, by way of example only, to the accompanying schematic drawings. It is important to note that the following schematic drawings are provided solely as exemplary, non-limiting potential embodiments of the invention. Accordingly, the drawings are to be regarded as illustrative instead of restrictive, serving merely to aid in the explanation of the invention's principles and potential applications.

Figures 1 and 2 show an example of relative-movement time measurements in stationary mode (Figure 1) and in pullback mode (Figure 2). It is noted that the drawings are schematic, and the blood vessels and the probes are not drawn at scale or in proportion, and their purpose is merely to illustrate how the measurements can be carried out to obtain the parameters necessary to calculate haemodynamic parameters, such as microvascular function parameters, wherein said parameters are preferably the absolute coronary flow, the coronary flow reserve and/or the microvascular resistance. The lengths and velocities are understood to be represented in absolute value, to not account for the directional negative or positive signs, for illustrative purpose and intuitive understanding.

The figures provided are merely representational and may not be drawn to scale. Certain proportions thereof may be exaggerated, while others may be minimized. The figures are intended to illustrate various implementations of the invention that can be understood and appropriately carried out by those of ordinary skill in the art. Commonly designated elements among the various figures refer to common or equivalent elements in the depicted embodiments. The figures are not intended to be exhaustive or to limit the invention to the precise form disclosed. It should be understood that the invention can be practiced with modification and alteration, and that the invention be limited only by the claims and the equivalents thereof.

## Claims

1. An Optical Coherence Tomography (OCT) system comprising an OCT probe (1) and a processing unit in communication with a memory and the OCT probe (1), wherein the OCT probe (1) is configured to be placed inside a blood vessel (3),
wherein the memory comprises instructions which, when executed by the processing unit, cause said processing unit to perform the following steps:
a) detect the time *t*₀ at which the distal end (21) of a first travelling column of an OCT-detectable liquid (ODL) (2) of volume *V_{c}* travelling inside the blood vessel (3) towards the OCT probe tip at a velocity *v_{c}* reaches a reference point (11) in the OCT probe (1), and the time *t*₁ at which the proximal end (22) of the first travelling column of ODL (2) reaches said reference point (11) in the OCT;
b) calculate a relative-movement time *T*₁ of the ODL column (2) with respect to the reference point (11) of the OCT probe (1) as a function of *t*₀ and *t*₁, preferably as the difference between *t*₁ and *t*₀; and
c) calculate one or more haemodynamic parameters associated to the blood vessel (3) as a function of *T*₁, preferably wherein said haemodynamic parameters comprise the absolute coronary flow, the coronary flow reserve and/or the microvascular resistance.

2. The OCT system according to claim 1, wherein the OCT probe (1) is configured to remain stationary inside the blood vessel (3) during the travelling of the first ODL column (2), and wherein the memory comprises further instructions which, when executed by the processing unit, cause said processing unit to:
d) calculate the absolute coronary flow Q₁ as a function of *V_{c}* and the relative-movement time *T*₁.

3. The OCT system according to claim 1 or 2, wherein the memory comprises further instructions which, when executed by the processing unit, cause said processing unit to:
e) perform steps (a) and (b) in hyperaemia conditions of the vessel (3) to calculate a hyperaemia relative-movement time *T*_{1*H*} of the ODL column (2) with respect to the OCT probe (1); and
f) estimate a coronary flow reserve (CFR) parameter as a function of *T*₁ and *T*₁*_{H},* preferably as the ratio between *T*_{1*H*} and *T*₁.

4. The OCT system according to claim 1, wherein the probe is further configured to be displaced at a pullback speed *PBs*₁ when the first column of ODL (2) is travelling inside the vessel (3), and at a pullback speed *PBs*₂*,* different than *PBs*₁, when a second column of ODL (2) of volume *V_{c}* is travelling inside the vessel (3) towards the OCT probe tip at a velocity *v_{c}*, wherein the volume *V_{c}* and the velocity *v_{c}* of the second column of ODL (2) are the same or approximately the same as those of the first column of ODL (2),
and wherein the memory comprises further instructions which, when executed by the processing unit, cause said processing unit to:
g) optionally, calculate a first probe displacement length ${l}_{1}^{′}$ as a function of *T*₁ and *PBs*₁;
h) detecting the time ${t}_{0}^{′}$ at which the distal end (21) of the second travelling column of ODL (2) reaches a reference point (11) in the OCT probe (1), and the time ${t}_{1}^{′}$ at which the proximal end (22) of the travelling column of ODL (2) reaches said reference point (11) in the OCT;
i) calculate a relative-movement time *T*₂ of said second column of ODL (2) with respect to the OCT probe (1) reference point (11) as a function of ${t}_{0}^{′}$ and ${t}_{1}^{′}$, and optionally calculate a second probe displacement length ${l}_{2}^{′}$ as a function of *T*₂ and *PBs₂*;
j) calculate the ODL column (2) velocity *v_{c}* and/or the column length *l_{c},* as a function of *T*₁, *T*₂*, PBs*₁ and *PBs₂,* or as a function of ${l}_{1}^{′} , {l}_{2}^{′}$ *PBs*₁ and *PBs₂*; and
k) calculate one or more haemodynamic parameters associated to the blood vessel (3) as a function of *v_{c}* and/or *l_{c},* preferably wherein said haemodynamic parameters are the absolute coronary flow, the coronary flow reserve and/or the microvascular resistance.

5. The OCT system according to claim 4, wherein the memory comprises further instructions which, when executed by the processing unit, cause said processing unit to:
l) calculate the ODL column (2) velocity in hyperaemia conditions *v_{cH}*; and
m) calculate the coronary flow reserve CFR as a function of *v_{cH}* and *v_{c}*, preferably as the ratio between *v_{cH}* and *v_{c}.*

6. The OCT system according to claim 4 or claim 5, wherein the memory comprises further instructions which, when executed by the processing unit, cause said processing unit to calculate the absolute coronary flows *Q*₁ and/or *Q*₂ of the first and second ODL columns (2) respectively, as a function of the volume *V_{c}* and the transit time *TT*₁ and/or *TT*₂ of the first and second ODL columns respectively, wherein *TT*₁ is calculated as a function of *PBs*₁, *v_{c}* and *T*₁, and *TT*₂ is calculated as a function of *PBs*₂*, v_{c}* and *T*₂.

7. The OCT system according to any one of claims 2 or 6 to 9, wherein the memory comprises further instructions which, when executed by the processing unit, cause said processing unit to:
n) image with the OCT probe (1) the blood vessel (3) and estimate the pressure P_{d} at a distal point of the vessel (3) by means of computational physiology, preferably by optical flow ratio (OFR); and
o) calculate a microvascular resistance parameter Rₘ as a function of the pressure P_{d} and the absolute coronary flow *Q*₁ or *Q*₂, or as a function of the pressure P_{d} and the transit time *TT*₁ or *TT*₂*.*

8. The OCT system according to any of the previous claims further comprising, or further configured to be connected to, an automatic injector of ODL, able to infuse a column of known volume *V_{c}* of ODL at a constant flow rate (*Qᵣ*) during a predetermined infusion time (*Tᵢ*).

9. The OCT system according to any one of the previous claims, further comprising a guide-extension catheter to inject the ODL column (2).

10. Computer program comprising instructions which, when executed by a processor, causes the processor to carry out the following steps:
(I) detecting the time *t*₀ at which the distal end (21) of a first travelling column of ODL (2) of known volume *V_{c}* travelling at a velocity *v_{c}* towards the tip of an OCT probe (1) placed inside a blood vessel (3), reaches a reference point (11) in the OCT probe (1), and the time *t*₁ at which the proximal end (22) of the first travelling column of ODL (2) reaches said reference point (11) in the OCT;
(II) calculating a relative-movement time *T*₁ of the ODL column (2) with respect to the reference point (11) of the OCT probe (1) as a function of *t*₀ and *t*₁, preferably as the difference between *t*₁ and *t*₀;
(III) calculating one or more haemodynamic parameters associated to the blood vessel (3) as a function of *T*₁, preferably wherein said haemodynamic parameters comprise the absolute coronary flow, the coronary flow reserve and/or the microvascular resistance, more preferably the haemodynamic parameters comprise the absolute coronary flow Q₁ calculated as a function of *V_{c}* and the relative-movement time *T*₁.

11. The computer program according to claim 10, comprising further instructions which, when executed by a processor, causes the processor to further carry out the following steps:
(IV) estimating a coronary flow reserve (CFR) parameter as a function of *T*₁ and *T*₁*_{H},* preferably as the ratio between *T*_{1*H*} and *T*₁, wherein *T*_{1*H*} is a hyperaemia relative-movement time *T*_{1*H*} of the ODL column (2) with respect to the reference point (11) of the OCT probe (1), obtained performing steps (i) to (iv) in hyperaemia conditions of the vessel (3).

12. The computer program according to any one of the previous claims, comprising further instructions which, when executed by a processor, causes the processor to carry out the following steps:
(V) detecting the times *t*₀ and ${t}_{0}^{′}$, respectively, at which the distal end (21) of the first and a second columns of ODL (2) both having each a volume *V_{c}* and both travelling inside a blood vessel (3) at a velocity *v_{c}*, reach the reference point (11) in the OCT probe (1), and the times *t*₁ and ${t}_{1}^{′}$ at which the proximal end (22) of the first and second travelling columns of ODL (2), respectively, reach said reference point (11) in the OCT, wherein the OCT probe (1) is moving at a pullback speed *PBs*₁ with respect to the first column of ODL (2) and at a pullback speed *PBs*₂ with respect to the second column of ODL (2);
(VI) calculating relative-movement times *T*₁ and *T*₂ of the first and second ODL columns (2), respectively, with respect to the reference point (11) of the OCT probe (1) as a function of *t*₀ and *t*₁, and as a function of ${t}_{0}^{′}$ and ${t}_{1}^{′}$, respectively, preferably as the difference between *t*₁ and *t*₀ and between ${t}_{1}^{′}$ and ${t}_{0}^{′}$;
(VII) optionally, calculating a first probe displacement length ${l}_{1}^{′}$ as a function of *T*₁ and *PBs*₁, and a second probe displacement length ${l}_{2}^{′}$ as a function of *T*₂ and *PBs*₂; and
(VIII) calculating the ODL column (2) velocity *v_{c}* as a function of *T*₁, *T*₂*, PBs*₁ and *PBs₂,* or as a function of ${l}_{1}^{′} , {l}_{2}^{′}$*, PBs*₁ and *PBs₂,* wherein the ODL column (2) velocity *v_{c}* is related to the blood flow rate.

13. The computer program according to claim 12, comprising further instructions which, when executed by a processor, causes the processor to further carry out the following steps:
(IX) calculating the ODL column (2) velocity in hyperaemia conditions *v_{cH}*;
(X) calculating the coronary flow reserve CFR as a function of *v_{cH}* and *v_{c}*, preferably as the ratio between *v_{cH}* and *v_{c}.*

14. The computer program according to claims 12 or 13, comprising further instructions which, when executed by a processor, causes the processor to further carry out the following steps:
(XI) calculating the absolute coronary flows *Q*₁ and/or *Q*₂ as a function of *V_{c}* and the transit time *TT*₁ of the first ODL column (2) for *Q*₁ or the transit time *TT*₂ of the second ODL column (2) for *Q*₂, wherein *TT*₁ is calculated as a function of *PBs*₁, *v_{c}* and *T*₁, and *TT*₂ is calculated as a function of *PBs*₂*, v_{c}* and *T*₂.

15. The computer program according to any one of claims 12 to 14, comprising further instructions which, when executed by a processor, causes the processor to further carry out the following steps:
(XII) imaging with the OCT probe (1) the blood vessel (3) and estimate the pressure P_{d} at a distal point of the vessel (3) by means of computational physiology, preferably by optical flow ratio (OFR); and
(XIII) calculating a microvascular resistance parameter Rₘ as a function of the pressure P_{d} and the absolute coronary flow *Q*₁ or *Q*₂, or as a function of the pressure P_{d} and the transit time *TT*₁ or *TT₂.*
